# EUROPÄISCHE PATENTSCHRIFT

(11) **EP 3 312 261 B1**
(45) Veröffentlichungstag und Bekanntmachung des Hinweises auf die Patenterteilung: **14.07.2021**
(21) Anmeldenummer: 17208134.1
(22) Anmeldetag: 30.10.2014
(51) Int. Cl.: C10G 70/04

(54) **VERFAHREN ZUR AUFTRENNUNG EINES KOHLENWASSERSTOFFGEMISCHS**
METHOD FOR SEPARATING A HYDROCARBON MIXTURE
PROCÉDÉ DE SÉPARATION D'UN MÉLANGE D'HYDROCARBURES

(30) Priorität: 14.11.2013 EP 13005355
(43) Veröffentlichungstag der Anmeldung: 25.04.2018
(62) Teilanmeldung aus: 14792462.5
(73) Patentinhaber: Linde GmbH, 82049 Pullach (DE)
(72) Erfinder: FRITZ, Helmut, 81375 München (DE); SINN, Tobias, 81545 München (DE)

(56) Entgegenhaltungen:
- WO-A1-2007/018506
- US-A- 5 372 009

## Beschreibung

Die Erfindung betrifft ein Verfahren gemäß dem Oberbegriff des Patentanspruchs 1.

### Stand der Technik

Verfahren und Vorrichtungen zum Dampfspalten (engl. Steam Cracking) von Kohlenwasserstoffen sind bekannt und beispielsweise im Artikel "Ethylene" in Ullmann's Encyclopedia of Industrial Chemistry, online seit 15. April 2007, DOI 10.1002/14356007.a10_045.pub2, beschrieben.

Dampfspaltverfahren werden im kommerziellen Maßstab in Rohrreaktoren durchgeführt, die grundsätzlich mit einer Vielzahl von Kohlenwasserstoffen und Kohlenwasserstoffgemischen von Ethan bis Gasöl bis zu einem Siedepunkt von typischerweise 600 °C (sogenannter Ofeneinsatz) bes chickt werden können. Unter gleichen oder vergleichbaren Spaltbedingungen (siehe hierzu unten) betriebene Reaktionsrohre oder Gruppen von Reaktionsrohren, gegebenenfalls aber auch unter einheitlichen Spaltbedingungen betriebene Rohrreaktoren insgesamt, werden nachfolgend jeweils als "Spaltöfen" bezeichnet. Ein Spaltofen ist im hier verwendeten Sprachgebrauch also eine zum Dampfspalten verwendete bauliche Einheit, die einen Ofeneinsatz gleichen oder vergleichbaren Spaltbedingungen aussetzt. Eine Anlage zum Dampfspalten kann einen oder mehrere derartiger Spaltöfen aufweisen.

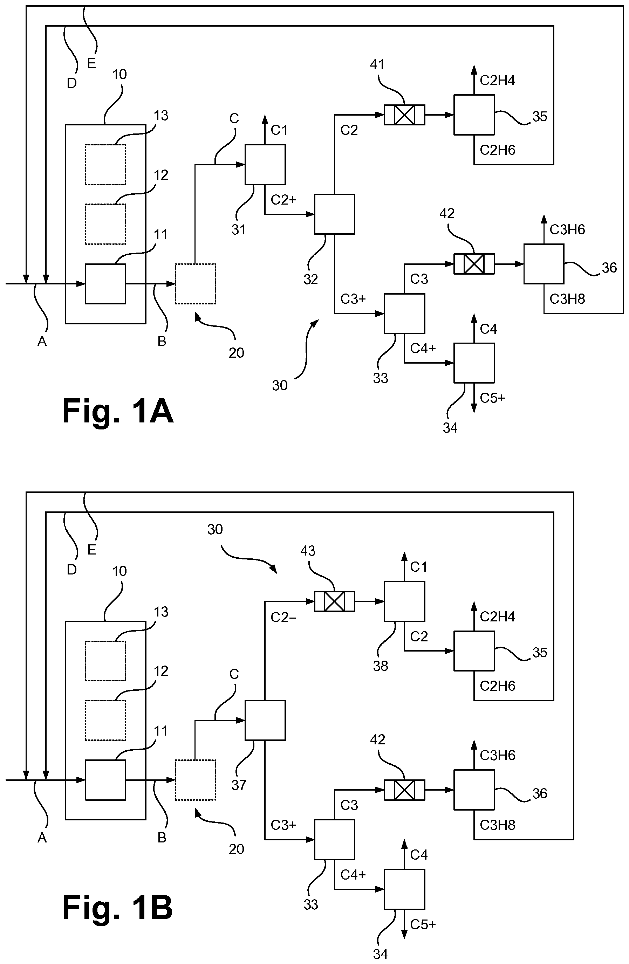

Der jeweilige Ofeneinsatz wird beim Dampfspalten in dem oder den Spaltöfen zumindest teilweise umgesetzt, wodurch ein sogenanntes Rohgas erhalten wird. Das Rohgas mehrerer Spaltöfen kann zusammengefasst und, wie auch unter Bezugnahme auf die Figuren 1A und 1B noch erläutert, einer Reihe von Nachbehandlungsschritten unterworfen werden. Derartige Nachbehandlungsschritte umfassen zunächst eine Aufbereitung des Rohgases, beispielsweise durch Quenchen, Kühlen und Trocknen, wodurch ein sogenanntes Spaltgas erhalten wird. Bisweilen wird auch das Rohgas als Spaltgas bezeichnet und umgekehrt.

Bei dem Spaltgas handelt es sich um ein Kohlenwasserstoffgemisch mit Kohlenwasserstoffen unterschiedlicher Kettenlänge und Struktur. Um aus dem Spaltgas die erwünschten Produkte zu gewinnen, muss dieses daher getrennt werden. Aus dem Stand der Technik sind hierzu unterschiedliche Verfahren bekannt und beispielsweise in dem erwähnten Artikel "Ethylene" in Ullmann's Encyclopedia of Industrial Chemistry ausführlich beschrieben. Verfahren zur Verarbeitung von Spaltgasen sind auch beispielsweise in der US 5,372,009 A und der US 5,361,589 A beschrieben.

Wie unten ebenfalls erläutert, setzt sich in einer Dampfspaltanlage der Ofeneinsatz eines oder mehrerer Spaltöfen typischerweise aus einem oder mehreren von extern zugeführten sogenannten Frischeinsätzen und aus einem oder mehreren aus dem Spaltgas abgetrennten Recycleströmen bzw. -fraktionen zusammen.

Die Zusammensetzung des Spaltgases ergibt sich unter anderem aus der Zusammensetzung des jeweils verwendeten Ofeneinsatzes.

In der WO 2007/018506 A1 wird ein Verfahren vorgeschlagen, das eine bestehende Olefinproduktionsanlage für die Gewinnung wenigstens eines gereinigten Ethylenprodukts aus einem gasförmigen Einsatzgemisch adaptiert. Die bestehende Olefinproduktionsanlage umfasst eine Demethanisierungsgruppe und eine Destillationsgruppe, die aus einem Ethylen und Ethan umfassenden Strom ein gereinigtes Ethylenprodukt abtrennt. Bei der Adaption wird eine Verteilerbatterie bereitgestellt, die einen Primärstrom, der etwa 15 bis 90 Prozent des Ethylens in dem Einsatzgemisch und Methan umfasst und im wesentlichen frei von Ethan ist, und einen oder mehrere Sekundärströme, die etwa 10 bis 85 Prozent des Ethylens in dem Einsatzgemisch und Ethan umfassen und im wesentlichen frei von Methan sind, aus dem Einsatzgemisch abtrennt. Der Primärstrom und der eine oder die mehreren Sekundärströme werden weiter aufgetrennt, wobei eine erstes und ein zweites gereinigtes Ethylenprodukt bereitgestellt werden.

Je ethanreicher der oder die die im Ofeneinsatz verwendeten Frischeinsätze sind, desto mehr Ethan enthält auch das Spaltgas. Somit finden sich im Spaltgas bei der Verwendung ethanreicher Kohlenwasserstoffgemische deutlich höhere Anteile an Ethan wieder als bei der Verwendung ethanarmer Kohlenwasserstoffgemische wie Naphtha.

Jedoch kann es aus wirtschaftlichen Gründen wünschenswert sein, ethanreiche Kohlenwasserstoffgemische einzusetzen. Diese fallen bei der Erdgasproduktion in großen Mengen an, unter anderem in Form sogenannter Erdgaskondensate (engl. Natural Gas Liquids, NGL), und können durch Dampfspalten zu Wertprodukten umgesetzt werden. Entsprechendes gilt auch für das durch Frackingverfahren geförderte vergleichsweise ethanreiche Schiefergas (engl. Shale Gas).

Eine Umstellung einer zur Verarbeitung ausschließlich ethanfreier Frischeinsätze wie Naphtha eingerichteten Dampfspaltanlage auf einen oder mehrere Frischeinsätze, die nicht nur unbeträchtliche Mengen an Ethan enthalten, ist nicht ohne weiteres möglich, weil der vergleichsweise hohe Ethananteil im Spaltgas bei der anschließenden Trennung nur mit großem baulichem Zusatzaufwand bewältigt werden kann. Beispielsweise muss hierbei eine Reihe der Trenneinheiten mehrfach bereitgestellt oder aufwendig in ihrer Kapazität erweitert werden.

Entsprechende Probleme treten selbstverständlich nicht nur dann auf, wenn herkömmliche Frischeinsätze wie beispielsweise Naphtha vollständig durch ethanreichere Frischeinsätze ersetzt werden, sondern auch bei einer nur teilweisen Verwendung ethanreicherer Frischeinsätze. Dies macht die gegebenenfalls erzielbaren wirtschaftlichen Vorteile wieder zunichte. Die Erfindung will hier Abhilfe schaffen.

### Offenbarung der Erfindung

Die genannte Aufgabe wird im Rahmen der vorliegenden Erfindung durch ein Verfahren mit den Merkmalen des Patentanspruchs 1 gelöst. Bevorzugte Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Mit dem bereits verwendeten Begriff "Ofeneinsatz" werden hier ein oder mehrere flüssige und/oder gasförmige Ströme bezeichnet, die einem oder mehreren Spaltöfen zugeführt werden. Auch durch ein entsprechendes Dampfspaltverfahren erhaltene Ströme, wie unten erläutert, können in einen oder mehrere Spaltöfen zurückgeführt und erneut als Ofeneinsatz verwendet werden. Als Ofeneinsatz eignet sich, wie erläutert, eine Vielzahl von Kohlenwasserstoffen und Kohlenwasserstoffgemischen von Ethan bis Gasöl bis zu einem Siedepunkt von typischerweise 600 °C. Wie erwähnt, betrifft die vorliegende Erfindung überwiegend die Verwendung von Ofeneinsätzen, die Frischeinsätze mit einem vergleichsweise hohen Gehalt an Ethan umfassen.

Ein solcher "Frischeinsatz" wird, wie erwähnt, von anlagenextern bereitgestellt und wird beispielsweise aus einer oder mehreren Erdölfraktionen, Erdgaskomponenten mit zwei bis vier Kohlenstoffatomen, darunter Ethan, und/oder Erdgaskondensaten gewonnen. Ein Ofeneinsatz kann auch aus einem oder mehreren sogenannten "Recycleströmen" bestehen, also Strömen, die in der Anlage selbst erzeugt und in einen entsprechenden Spaltofen zurückgeführt werden. Ein Ofeneinsatz kann auch aus einem Gemisch eines oder mehrerer Frischeinsätze mit einem oder mehreren Recycleströmen bestehen.

Eine "Dampfspaltanlage" umfasst gemäß dem hier verwendeten Sprachgebrauch einen oder mehrere Spaltöfen, die bei gleichen oder unterschiedlichen Spaltbedingungen betrieben und mit gleichen oder unterschiedlichen Ofeneinsätzen beschickt werden können, sowie eine zur Trennung eines erhaltenen Spaltgases eingerichtete "Trennanlage", die typischerweise eine Reihe von Destillationssäulen umfasst und zur Trennung des Spaltgases in mehrere Fraktionen auf Grundlage der Siedepunkte der enthaltenen Kohlenwasserstoffe eingerichtet ist.

In der Fachwelt werden für entsprechende Fraktionen Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (jedoch konventionsgemäß unter Umständen auch Wasserstoff, dann auch "C1 minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Dies gilt auch für eine "C5-Fraktion" und die höheren Fraktionen. Mehrere solcher Fraktionen können auch verfahrens- und/oder bezeichnungsmäßig zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

Flüssige und gasförmige Ströme können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Der Begriff "überwiegend" bezeichnet einen Gehalt von wenigstens 50%, 60%, 70%, 80% oder 90% oder entspricht dem Begriff "reich". Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält.

Enthält ein Frischeinsatz im hier verwendeten Sprachgebrauch Ethan "in nicht nur unbeträchtlicher Menge", so weist dieser einen Ethangehalt auf, der beispielsweise mehr als 5%, 10%, 20%, 30% oder 40% beträgt. Grundsätzlich könnte auch reines Ethan als Frischeinsatz verwendet werden, beispielsweise zusammen mit anderen schwereren Frischeinsätzen wie Naphtha. Typischerweise liegt die Obergrenze des Ethangehalts entsprechender Frischeinsätze jedoch bei 90%, 80%, 70%, 60% oder 50%. Beispielsweise handelt es sich bei Schiefergas oder Erdgaskondensaten um Frischeinsätze, die Ethan in nicht nur unbeträchtlicher Menge enthalten.

Prozentangaben können im Rahmen dieser Anmeldung Volumenprozent, Molprozent oder Masseprozent darstellen. Drücke werden als Absolutdrücke angegeben.

Ein Strom kann von einem anderen Strom "abgeleitet" sein, beispielsweise durch Verdünnung, Aufkonzentrierung, Anreicherung, Abreicherung, Trennung oder Umsetzung beliebiger Komponenten, durch Trennschritte oder auch durch eine Vereinigung mit wenigstens einem weiteren Strom. Ein abgeleiteter Strom kann auch durch Aufteilen eines Ausgangsstroms in wenigstens zwei Teilströme gebildet werden, wobei dann jeder Teilstrom oder auch ein Reststrom nach Abtrennung eines anderen Stroms ein solcher abgeleiteter Strom ist.

### Vorteile der Erfindung

Die vorliegende Erfindung geht von einem bekannten Verfahren aus, bei dem zumindest teilweise durch Dampfspalten ein Kohlenwasserstoffgemisch gewonnen und getrennt wird. Ein derartiges Kohlenwasserstoffgemisch wird, wie zuvor erläutert, als Spalt- oder Rohgas bezeichnet und wird einem oder mehreren Aufbereitungsschritten unterworfen. Das Kohlenwasserstoffgemisch weist zumindest Kohlenwasserstoffe mit einem, zwei und drei Kohlenstoffatomen, darunter Ethan und Ethylen auf. Wie erläutert, sind die Ethanmengen in einem derartigen Spaltgas bei der Verwendung von Frischeinsätzen, die Ethan in nicht nur unbeträchtlicher Menge enthalten, beim Dampfspalten erhöht.

Selbst wenn überwiegend Ethan enthaltende Kohlenwasserstoffgemische als Ofeneinsätze für das Dampfspalten verwendet werden, enthält das Spaltgas Kohlenwasserstoffe mit mehr als drei Kohlenstoffatomen. Wie erwähnt, werden herkömmliche Frischeinsätze wie Naphtha auch nicht notwendigerweise vollständig durch ethanreiche Frischeinsätze ersetzt, so dass auch aus diesem Grund typischerweise beträchtliche Mengen an Kohlenwasserstoffe mit mehr als drei Kohlenstoffatomen im Spaltgas enthalten sind.

Die vorzunehmende Trennung unterscheidet sich damit nicht notwendigerweise qualitativ, jedoch sehr wohl quantitativ (d.h. in Bezug auf die jeweiligen Mengenanteile der zu trennenden Fraktionen), wenn Frischeinsätze, die Ethan in nicht nur unbeträchtlicher Menge enthalten, beim Dampfspalten eingesetzt werden.

Aus dem Kohlenwasserstoffgemisch wird zunächst unter zumindest teilweiser Abtrennung anderer Komponenten eine Fraktion gewonnen, die hier als "erste Fraktion" bezeichnet wird. Wie auch nachfolgend noch erläutert, unterscheidet man im Stand der Technik zwischen sogenannten "Demethanizer First"- und "Deethanizer First"-Verfahren. Diese umfassen, in allgemeiner Formulierung, dass aus dem Kohlenwasserstoffgemisch zunächst unter zumindest teilweiser Abtrennung anderer Komponenten eine "erste" Fraktion gewonnen wird, die, wie erfindungsgemäß vorgesehen, entweder den überwiegenden Teil der in dem Kohlenwasserstoffgemisch zuvor enthaltenen Kohlenwasserstoffe mit zwei und mehr Kohlenstoffatomen oder aber, wie nicht erfindungsgemäß vorgesehen, den überwiegenden Teil der in dem Kohlenwasserstoffgemisch zuvor enthaltenen Kohlenwasserstoffe mit zwei und weniger Kohlenstoffatomen enthält.

Den überwiegenden Anteil der in dem Kohlenwasserstoffgemisch enthaltenen Kohlenwasserstoffe mit zwei und mehr Kohlenstoffatomen enthält diese erste Fraktion dann, wenn sie zuvor einen sogenannten Demethanizer durchlaufen hat, d.h. leichtere Komponenten (Methan und gegebenenfalls Wasserstoff) abgetrennt wurden. Werden hingegen in einem Deethanizer zunächst Kohlenwasserstoffe mit drei oder mehr Kohlenstoffatomen aus einem entsprechenden Kohlenwasserstoffgemisch abgetrennt, verbleibt eine erste Fraktion, die den überwiegenden Teil der im Kohlenwasserstoffgemisch zuvor enthaltenem Kohlenwasserstoffe mit zwei und weniger Kohlenstoffatomen enthält.

Das erfindungsgemäße Verfahren eignet sich für Demethanizer First-Verfahren und entsprechende Varianten solcher Verfahren, wie sie allgemein bekannt sind, weil die hier als "erste Fraktion" bezeichnete Fraktion, die stromab des Trennprozesses (Demethanizer) vorliegt, jeweils die gesamte oder den überwiegenden Anteil der im ursprünglichen Kohlenwasserstoffgemisch (Spalt- bzw. Rohgas) enthaltenen Ethanmenge enthält. Wie mehrfach erläutert, ist diese Ethanmenge bei der Verwendung von Frischeinsätzen, die Ethan in nicht nur unbeträchtlicher Menge enthalten, deutlich erhöht.

Sowohl bei Demethanizer First- als auch bei Deethanizer First-Verfahren werden aus der erläuterten ersten Fraktion anschließend weitere Fraktionen gewonnen. Bei diesen weiteren Fraktionen kann es sich um Fraktionen mit Kohlenwasserstoffen mit unterschiedlicher und/oder einheitlicher Kettenlänge und/oder gleicher Summen- und/oder Strukturformel handeln. Ein typisches Beispiel für entsprechende weitere Fraktionen sind beispielsweise Ethylen und/oder Butadien und/oder Fraktionen mehrerer Kohlenwasserstoffe mit unterschiedlicher und/oder einheitlicher Kettenlänge, die in aus einer entsprechenden Anlage ausgeleitet und/oder in einen Dampfspaltprozess zurückgeführt werden (sogenannte Recyclefraktionen). Eine solche Recyclefraktion ist erfindungsgemäß aus dem Spaltgas abgetrenntes Ethan.

Wie ebenfalls mehrfach erläutert, sind gegebenenfalls Trennanlagen, die als Teil von Dampfspaltanlagen zur Bearbeitung ethanarmer Frischeinsätze ausgebildet sind, häufig nicht dafür eingerichtet, die bei der Verarbeitung von ethanreicheren Frischeinsätzen höheren Ethanmengen im erhaltenen Kohlenwasserstoffgemisch (Spalt- bzw. Rohgas) kapazitiv oder trenntechnisch zu bewältigen.

Erfindungsgemäß ist vorgesehen, nach der zumindest teilweisen Abtrennung der anderen Komponenten aus dem ursprünglichen Kohlenwasserstoffgemisch, also nach der Bildung der ersten Fraktion, insbesondere stromab eines Demethanizers auch eine Ethan enthaltene Fraktion abzutrennen, so dass sich der Gehalt an Ethan in der ersten Fraktion auf weniger als 25% verringert. Die Abtrennung erfolgt vor der anschließenden Gewinnung der weiteren Fraktionen. Erfolgt die Abtrennung stromab der zumindest teilweisen Abtrennung der anderen Komponenten aus dem ursprünglichen Kohlenwasserstoffgemisch, wird die erste Fraktion in ihrem Ethangehalt reduziert. Auf diese Weise kann der Gehalt an Ethan in der ersten Fraktion beispielsweise an die bei der Verwendung ethanarmer Frischeinsätze erhaltenen Ethangehalte angepasst werden. Dies ermöglicht es, bei der Umstellung des oder der Frischeinsätze die Trennanlage bis auf die erfindungsgemäß vorgesehenen Maßnahmen weitgehend unverändert weiter zu betreiben.

Mit anderen Worten wird mittels der erfindungsgemäßen Abtrennung der Ethan enthaltenden Fraktion der Ethangehalt der ersten Fraktion soweit verringert, dass auch bestehende Trennanlagen zu deren Bearbeitung befähigt sind. Im Gegensatz zur Vergrößerung der Kapazität der nachgeordneten Trenneinheiten zur Gewinnung der weiteren Fraktionen, die anderenfalls zur Bewältigung der erhöhten Ethanmenge erforderlich wäre, ermöglicht das erfindungsgemäße Verfahren eine vergleichsweise einfache und kostengünstige Reduktion erhöhter Ethangehalte.

Die Ethan enthaltende Fraktion enthält neben Ethan ggf. weitere Komponenten, die sich nach dem Verfahren, das zur Gewinnung der ersten Fraktion verwendet wird, richten. So kann eine derartige Ethan enthaltende Fraktion beispielsweise Kohlenwasserstoffe mit drei und mehr Kohlenstoffatomen enthalten, wenn ein Demethanizer First-Verfahren zum Einsatz kommt, wie es erfindungsgemäß der Fall ist. Die Ethan enthaltende Fraktion kann auch überwiegend (im Sinne der obigen Definition) aus Ethan bestehen.

Die überwiegend Ethan enthaltende Fraktion ist jedenfalls im obigen Sinn arm an anderen Kohlenwasserstoffen mit zwei Kohlenstoffatomen, vorzugsweise frei von diesen. Solche anderen Kohlenwasserstoffe mit zwei Kohlenstoffatomen werden in nachgeschalteten Trennschritten aus der ersten Fraktion gewonnen. Beispielsweise ist Ethylen zu höchstens 1,5%, 1,0%, 5000 ppm, 4000 ppm oder 3000 ppm enthalten.

Ein erfindungsgemäßes Verfahren umfasst insbesondere, einen Gehalt an Ethan vorzugeben, der in einer zur Durchführung des Verfahrens verwendeten Trennanlage zulässig ist. Bei einer entsprechenden Vorgabe können auch die Werte verwendet werden, die typischerweise bei der Bearbeitung ethanarmer Frischeinsätze entstehen. Die Menge der Ethan enthaltenden Fraktion wird in Entsprechung hieran angepasst. Es wird also, wie erläutert, die Menge des Ethans in der ersten Fraktion so weit reduziert, dass der tolerierbare Ethangehalt in den nachgeordneten Einrichtungen bzw. Trenneinheiten eingehalten werden kann. Hierbei handelt es sich beispielsweise auch um einen Ethangehalt von weniger als 20%, 17%, 15%, 13% oder 10%.

Der Gehalt an Ethan in der ersten Fraktion nach der Abtrennung der überwiegend Ethan enthaltenden Fraktion definiert auch deren Gesamtmenge bzw. deren Volumenstrom, so dass sich eine hier "zulässige" Ethanmenge auch beispielsweise aus den entsprechenden hydraulischen Limitierungen bestehender Bauteile ergibt. Auch sind beispielsweise Hydriereinrichtungen, wie sie in den beigefügten Figuren dargestellt sind, in ihrem maximalen Durchsatz limitiert, so dass auch hier eine maximal zulässige Ethanmenge (die die Menge bzw. den Volumenstrom des jeweiligen Stroms definiert) nicht überschritten werden darf.

Wie erläutert, kann die Ethan enthaltende Fraktion insbesondere noch weitere Kohlenwasserstoffe enthalten, insbesondere Spuren an Ethylen und Acetylen sowie ggf. Kohlenwasserstoffe mit drei und mehr Kohlenstoffatomen. Diese können aus der Ethan enthaltenden Fraktion abgetrennt werden, so dass anschließend eine reine Ethanfraktion bzw. eine ethanreiche Fraktion vorliegt.

Vorteilhaft ist es, die Ethan enthaltende Fraktion zumindest teilweise durch Dampfspalten zu verarbeiten, was bedeutet, dass entsprechendes Ethan als Recyclestrom durch die verwendete Dampfspaltanlage geführt wird.

Wie bereits erläutert, eignet sich das erfindungsgemäße Verfahren für Demethanizer First-Verfahren. Bei einem Demethanizer First-Verfahren wird, wie zuvor erläutert, zur Gewinnung der ersten Fraktion aus dem Kohlenwasserstoffgemisch eine Fraktion abgetrennt, die den überwiegenden Anteil des in diesem enthaltenen Methans und Wasserstoffs enthält (es wird also eine sogenannte C1minus-Fraktion abgetrennt). Die erste Fraktion enthält hierdurch den überwiegenden Anteil der in dem Kohlenwasserstoffgemisch enthaltenen Kohlenwasserstoffe mit zwei und mehr Kohlenstoffatomen.

Eine entsprechende Trennanlage kann eine erste Trenneinheit aufweisen, die dafür eingerichtet ist, aus dem Kohlenwasserstoffgemisch zunächst unter zumindest teilweiser Abtrennung anderer Komponenten eine erste Fraktion zu gewinnen, die den überwiegenden Teil der zuvor in dem Kohlenwasserstoffgemisch enthaltenen Kohlenwasserstoffe mit zwei und mehr Kohlenstoffatomen enthält. Es können weitere Trenneinheiten vorgesehen sein, die dafür eingerichtet sind, aus der ersten Fraktion anschließend weitere Fraktionen zu gewinnen, darunter Ethan, das zum Dampfspalten zurückgeführt wird, und Ethylen. Die Trennanlage kann eine zusätzliche Trenneinheit aufweisen, die zur Abtrennung einer Ethan enthaltenden Fraktion nach der zumindest teilweisen Abtrennung der anderen Komponenten und vor der Gewinnung der weiteren Fraktionen aus dem Kohlenwasserstoffgemisch in einer Menge eingerichtet ist, die den Gehalt an Ethan in der ersten Fraktion auf weniger als 25% verringert. Die Eigenschaften dieser überwiegend Ethan enthaltenden Fraktion wurden bereits ausführlich erläutert.

Die zusätzliche Trenneinheit kann als Destillationssäule ausgebildet sein, die vorzugsweise 60 bis 120, beispielsweise 70 bis 100, insbesondere 80 bis 90 Trennböden aufweist. Eine solche Dimensionierung eignet sich insbesondere für ein Demethanizer-First-Verfahren. Die vorteilhafterweise verwendeten Betriebsdrücke und Betriebstemperaturen richten sich nach dem Prozessumfeld und der Einbindestelle in die jeweilige Zerlegungssequenz.

Vorteilhafterweise ist eine solche Trennanlage zur Durchführung eines zuvor erläuterten Verfahrens eingerichtet.

Die Erfindung wird nachfolgend unter Bezugnahme auf die beigefügten Figuren gegenüber dem Stand der Technik und gegenüber nicht erfindungsgemäßen Vergleichsbeispielen erläutert.

### Kurze Beschreibung der Zeichnungen

Figur 1A veranschaulicht schematisch den Ablauf eines Verfahrens zur Erzeugung von Kohlenwasserstoffprodukten gemäß dem Stand der Technik.
Figur 1B veranschaulicht schematisch den Ablauf eines Verfahrens zur Erzeugung von Kohlenwasserstoffprodukten gemäß dem Stand der Technik.
Figur 2 veranschaulicht schematisch den Ablauf eines Verfahrens zur Trennung eines Kohlenwasserstoffgemischs gemäß einer Ausführungsform der Erfindung.
Figur 3 veranschaulicht schematisch den Ablauf eines Verfahrens zur Trennung eines Kohlenwasserstoffgemischs gemäß einer Ausführungsform der Erfindung.
Figur 4 veranschaulicht schematisch den Ablauf eines Verfahrens zur Trennung eines Kohlenwasserstoffgemischs gemäß einem nicht erfindungsgemäßen Vergleichsbeispiel.
Figur 5 veranschaulicht schematisch den Ablauf eines Verfahrens zur Trennung eines Kohlenwasserstoffgemischs gemäß einem nicht erfindungsgemäßen Vergleichsbeispiel.

In den Figuren sind einander entsprechende Elemente mit identischen Bezugszeichen versehen und werden der Übersichtlichkeit halber nicht wiederholt erläutert.

### Ausführliche Beschreibung der Zeichnungen

In Figur 1A ist der Ablauf eines Verfahrens zur Erzeugung von Kohlenwasserstoffprodukten durch Dampfspalten und anschließende Trennung eines erhaltenen Spaltgases in Fraktionen gemäß dem Stand der Technik in Form eines schematischen Ablaufplans dargestellt.

Kern des Verfahrens ist ein Dampfspaltprozess 10, der unter Verwendung eines oder mehrerer Spaltöfen 11 bis 13 durchgeführt werden kann. Nachfolgend wird nur der Betrieb des Spaltofens 11 erläutert, die weiteren Spaltöfen 12 und 13 können in entsprechender Weise arbeiten.

Der Spaltofen 11 wird mit einem Strom A als Ofeneinsatz beschickt, bei dem es sich teilweise um einem sogenannten Frischeinsatz handeln kann, der aus anlagenexternen Quellen zur Verfügung gestellt wird, und zu einem Teil um einen sogenannten Recyclestrom, der in dem Verfahren selbst gewonnen wird. Auch die anderen Spaltöfen 12 und 13 können mit entsprechenden Strömen beschickt werden. Unterschiedliche Ströme können auch in unterschiedliche Spaltöfen 11 bis 13 eingespeist werden, ein Strom kann auf mehrere Spaltöfen aufgeteilt werden oder mehrere Teilströme können zu einem Sammelstrom vereinigt werden, der beispielsweise als Strom A einem der Spaltöfen 11 bis 13 zugeführt wird.

Durch das Dampfspalten in dem Dampfspaltprozess 10 wird ein Rohgasstrom B erhalten, der bisweilen bereits an dieser Stelle als Spaltgasstrom bezeichnet wird. Der Rohgasstrom B wird in einer Reihe nicht dargestellter Aufbereitungsstufen eines Aufbereitungsprozesses 20 aufbereitet, beispielsweise einem sogenannten Ölquench unterzogen, vorfraktioniert, verdichtet, weiter gekühlt und getrocknet.

Der entsprechend behandelte Strom B, das eigentliche Spaltgas C und damit das im Rahmen der vorliegenden Erfindung getrennte Kohlenwasserstoffgemisch, wird anschließend einem Trennprozess 30 unterworfen. Der Trennprozess 30 ist in einer entsprechenden Trennanlage implementiert. Es wird eine Anzahl von Fraktionen gewonnen, die hier, wie eingangs erläutert, entsprechend der Kohlenstoffanzahl der überwiegend enthaltenen Kohlenwasserstoffe bezeichnet werden. Der in der Figur 1A dargestellte Trennprozess 30 arbeitet nach dem Prinzip "Demethanizer First", ein weiterer Trennprozess nach dem Prinzip "Deethanizer First" ist in Figur 1B dargestellt.

In dem Trennprozess 30 wird aus dem Spaltgas C zunächst in einer ersten Trenneinheit 31 (dem sogenannten Demethanizer) eine C1- bzw. C1 minus-Fraktion (mit dem Bezugszeichen C1 bezeichnet) gasförmig abgetrennt, die, falls nicht bereits zuvor entfernt, auch noch Wasserstoff enthalten kann. Sie wird typischerweise als Brenngas verwendet. Es verbleibt eine flüssige C2plus-Fraktion (Bezugszeichen C2+), die in eine zweite Trenneinheit 32 (den sogenannten Deethanizer) überführt wird. Die C2plus-Fraktion wird im Rahmen dieser Anmeldung als "erste" Fraktion bezeichnet, wenn ein Demethanizer First-Verfahren zum Einsatz kommt.

In dieser zweiten Trenneinheit 32 wird eine C2-Fraktion (Bezugszeichen C2) gasförmig aus der C2plus-Fraktion abgetrennt. Die C2plus-Fraktion kann beispielsweise einem Hydrotreatmentprozess 41 unterzogen werden, um enthaltenes Acetylen zu Ethylen umzusetzen. Anschließend wird die C2-Fraktion in einer C2-Trenneinheit 35 (auch als C2-Splitter bezeichnet) in Ethylen (Bezugszeichen C2H4) und Ethan (Bezugszeichen C2H6) aufgetrennt. Letzteres kann als Recyclestrom D in einem oder mehreren Spaltöfen 11 bis 13 erneut dem Dampfspaltprozess 10 unterworfen werden. Im dargestellten Beispiel werden die Recycleströme D und E zu dem Strom A zugegeben. Die Recycleströme D und E und der Strom A können auch in unterschiedliche Spaltöfen 11 bis 13 geführt werden.

Wie erläutert, erhöht sich beim Einsatz von Frischeinsätzen, die Ethan in nicht nur unbeträchtlicher Menge enthalten, insbesondere der Anteil des Ethans im Spaltgas C. Die erläuterten Trenneinheiten sind in bestehenden Anlagen, die für die Verwendung von ausschließlich ethanarmen Frischeinsätzen eingerichtet sind, nicht für entsprechend große Ethanmengen ausgelegt.

In der zweiten Trenneinheit 32 verbleibt eine flüssige C3plus-Fraktion (Bezugszeichen C3+), die in eine dritte Trenneinheit 33 (den sogenannten Depropanizer) überführt wird. In dieser wird aus der C3plus-Fraktion eine C3-Fraktion (Bezugszeichen C3) abgetrennt und beispielsweise einem weiteren Hydrotreatmentprozess 42 unterzogen, um Methylacetylen in der C3-Fraktion zu Propylen umzusetzen. Anschließend wird die C3-Fraktion in einer C3-Trenneinheit 36 in Propen (Bezugszeichen C3H6) und Propan (Bezugszeichen C3H8) aufgetrennt. Letzteres kann als Recyclestrom E in einem oder mehreren Spaltöfen 11 bis 13, separat oder mit anderen Strömen, erneut dem Dampfspaltprozess 10 unterworfen werden. In der dritten Trenneinheit 33 verbleibt eine flüssige C4plus-Fraktion (Bezugszeichen C4+), die in eine vierte Trenneinheit 34 (den sogenannten Debutanizer) überführt wird. In dieser wird aus der C4plus-Fraktion eine C4-Fraktion (Bezugszeichen C4) abgetrennt. Es verbleibt eine flüssige C5plus-Fraktion (Bezugszeichen C5+).

Bei der Verwendung von ausschließlich gasförmigen Ofeneinsätzen fallen gegebenenfalls keine oder deutlich geringere Mengen an C3plus-, C4plus- oder C5plus-Kohlenwasserstoffen an, so dass auf die zuletzt genannten Trenneinheiten verzichtet werden kann.

Es versteht sich, dass sämtliche der dargestellten Fraktionen auch geeigneten Nachbehandlungsschritten unterworfen werden können. Beispielsweise kann aus dem C4-Kohlenwasserstoffstrom, falls gewonnen, 1,3-Butadien abgetrennt werden. Es können ferner zusätzliche Recycleströme verwendet werden, die analog zu den Recycleströmen D und E dem Dampfspaltprozess 10 unterworfen werden können.

In Figur 1B ist der Ablauf eines alternativen Verfahrens zur Erzeugung von Kohlenwasserstoffen durch Dampfspalten gemäß dem Stand der Technik in Form eines schematischen Ablaufplans dargestellt. Auch hier ist Kern des Verfahrens ein Dampfspaltprozess 10, der unter Verwendung eines oder mehrerer Spaltöfen 11 bis 13 ablaufen kann. Im Gegensatz zu dem Verfahren der Figur 1A wird hier das Spaltgas C einem Trennprozess 30 nach dem Prinzip "Deethanizer First" unterworfen.

In dem Trennprozess 30 wird dabei aus dem Spaltgas C zunächst in einer ersten Trenneinheit 37 eine C2minus-Fraktion (Bezugszeichen C2-) gasförmig abgetrennt, die überwiegend Methan, Ethan, Ethylen und Acetylen und, falls nicht bereits zuvor entfernt, auch noch Wasserstoff enthalten kann. Die C2minus-Fraktion wird im Rahmen dieser Anmeldung als "erste" Fraktion bezeichnet, wenn ein Deethanizer First-Verfahren zum Einsatz kommt.

Die C2minus-Fraktion kann insgesamt einem Hydrotreatmentprozess 43 unterzogen werden, um enthaltenes Acetylen zu Ethylen umzusetzen. Anschließend wird aus der C2minus-Fraktion in einer C2minus-Trenneinheit 38 eine C1-Fraktion abgetrennt und wie oben weiter verwendet. Es verbleibt eine C2-Fraktion, die in einer C2-Trenneinheit 35 wie oben in Ethylen und Ethan aufgetrennt wird. Letzteres kann auch hier als Recyclestrom D in einem oder mehreren Spaltöfen 11 bis 13 erneut dem Dampfspaltprozess 10 unterworfen werden. In der ersten Trenneinheit 37 verbleibt auch hier gegebenenfalls eine flüssige C3plus-Fraktion, die in den Trenneinheiten 33 bis 36 und gegebenenfalls der Hydrotreatmenteinheit 42, wie bereits zu Figur 1 erläutert, behandelt wird.

Auch hier stellt ein erhöhter Anteil von Ethan im Spaltgas ein Problem dar, da die erläuterten Trenneinheiten in Anlagen für ausschließlich ethanarme Frischeinsätze nicht für entsprechend große Ethanmengen ausgelegt sind.

Dem Fachmann sind, beispielsweise aus dem eingangs erwähnten Artikel "Ethylene" in Ullmann's Encyclopedia of Industrial Chemistry, mehrere weitere

Verfahrensalternativen bekannt, die sich insbesondere in der Aufbereitung des Spaltgases C und/oder dem verwendeten Trennprozess unterscheiden.

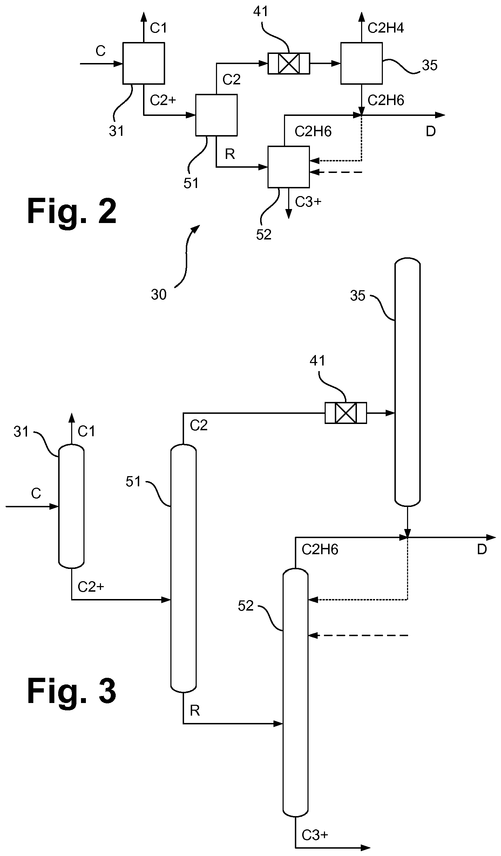

Figur 2 zeigt einen Ansatz zur Lösung des Problems des erhöhten Anteils des Ethans im Spaltgas bei der Verwendung von Frischeinsätzen, die Ethan in nicht nur unbeträchtlicher Menge enthalten, gemäß einer Ausführungsform der Erfindung.

Das in Figur 2 gezeigte Verfahren ist in Form eines schematischen Ablaufplans dargestellt und beruht auf dem in Figur 1A gezeigten Verfahren nach dem Prinzip Demethanizer First. Zur Veranschaulichung der universellen Einsetzbarkeit des Verfahrens gemäß der gezeigten Ausführungsform wurde auf die zur Herstellung des Spaltgases C verwendeten Prozesse bzw. Vorrichtungen verzichtet.

Ein entsprechendes Spaltgas C kann jedoch in analoger Weise zu dem beispielsweise in Figur 1A gezeigten Verfahren gewonnen werden. Insbesondere stammt ein derartiges Spaltgas C aus einem oder mehreren Spaltöfen 11 bis 13, die zumindest teilweise mit einem Frischeinsatz als Ofeneinsatz beschickt werden, der Ethan in nicht nur unbeträchtlicher Menge aufweist.

Wie erläutert, kommt es bei Verwendung ausschließlich gasförmiger Ofeneinsätze gegebenenfalls zur Bildung keiner oder deutlich geringerer Mengen an C3plus-, C4plus- oder C5plus-Kohlenwasserstoffen, so dass auf eine Darstellung der zur Trennung entsprechender Fraktionen verwendeten Prozesse bzw. Vorrichtungen verzichtet wurde. Fallen entsprechende Kohlenwasserstoffe an, können auch in dem in Figur 2 gezeigten Verfahren solche Prozesse bzw. Vorrichtungen vorgesehen sein.

Anstelle eines Spaltgases C kann auch beispielsweise eine Fraktion eines entsprechenden Spaltgases verwendet werden, die aus einer Trennung in Kohlenwasserstoffe mit vier und mehr Kohlenstoffatomen einerseits und Kohlenwasserstoffe mit drei und weniger Kohlenstoffatomen andererseits resultiert.

Das in Figur 2 dargestellte Verfahren gemäß einer Ausführungsform der Erfindung unterscheidet sich von dem in Figur 1A gezeigten Verfahren insbesondere durch die Verwendung einer zusätzlichen Trenneinheit 51 in dem Trennprozess 30.

In der zusätzlichen Trenneinheit 51, beispielsweise einer Destillationssäule, die die oben angegebenen Merkmale aufweist, wird eine flüssige, Ethan enthaltende Fraktion abgetrennt, die jedoch bei einem Demethanizer First-Verfahren, wie es hier dargestellt ist, noch zusätzliche schwerer siedende Komponenten, insbesondere C3plus- und höhere Kohlenwasserstoffe, enthält. Der Gehalt richtet sich, wie erwähnt, nach dem Ofeneinsatz und ist bei ausschließlich gasförmigen Ofeneinsätzen relativ gering. Diese überwiegend Ethan enthaltende Fraktion trägt in der Figur 2 das Bezugszeichen R.

Diese Fraktion R, die überwiegend Ethan und die schwerer siedenden Komponenten enthält, ist gleichzeitig arm an anderen Kohlenwasserstoffen mit zwei Kohlenstoffatomen. Sie wird anschließend in eine weitere Trenneinheit 52 überführt, in der die schwerer siedenden C3plus-Kommponenten und das Ethan voneinander getrennt werden. Der Trenneinheit 52 kann auch ein Teil des Ethans bzw. ein Teil einer entsprechenden ethanreichen Fraktion aus der zuvor erläuterten Trenneinheit 35 (punktiert dargestellt) zugeführt werden. Hierdurch lässt sich Energie einsparen und die ohnehin vorhandene Trennkapazität der Trenneinheit 52 nutzen. In der Trenneinheit 52, die typischerweise als Destillationssäule ausgebildet ist, kann diese Ethanfraktion aus der Trenneinheit 35 insbesondere am Kopf aufgegeben werden. Ferner kann, wie mit einem gestrichelten Pfeil veranschaulicht, weiteres Ethan eingespeist werden.

Eine in der Trenneinheit 52 erhaltene Ethanfraktion, beispielsweise ein Kopfprodukt einer entsprechenden Destillationssäule, kann abgezogen, mit weiteren Strömen vereinigt, und in einen Spaltprozess zurückgeführt werden, wie bereits zuvor mit Strom D veranschaulicht.

Bei dem weiteren in der Trenneinheit 51 erhaltenen Strom, der hier mit C2 bezeichnet ist, handelt es sich um den nach der Abtrennung der überwiegend Ethan enthaltenden Fraktion R (mit den C3plus-Kohlenwasserstoffen) verbleibenden Anteil der C2plus-Fraktion, der durch die Abtrennung auf jeden beliebigen Ethangehalt eingestellt werden kann. Damit brauchen die stromab der Trenneinheit 51 angeordneten Trenneinheiten (wie in Figur 1A gezeigt) bei der Umstellung einer entsprechenden Spaltanlage von ethanarmen auf ethanreiche(re) Frischeinsätze nicht verändert zu werden.

Die Funktion der zusätzlichen Trenneinheit 51 entspricht damit teilweise der Funktion der Trenneinheit 32 eines herkömmlichen Verfahrens (vgl. Figur 1A), wobei jedoch in der Trenneinheit 51 keine "reine" C3plus-Fraktion sondern eine C3plus-Fraktion mit einem nicht unbeträchtlichen Anteil an Ethan (nämlich die mehrfach erwähnte Ethan enthaltende Fraktion) abgetrennt wird. Auch die Funktion der Trenneinheit 52 entspricht teilweise der Funktion der Trenneinheit 32 des herkömmlichen Verfahrens, allerdings mit dem Unterschied, dass in der Trenneinheit 52 vergleichsweise reines Ethan von den C3plus-Kohlenwasserstoffen getrennt wird. Es handelt sich funktionell um einen sogenannten Deethanizer, der ohnehin vorhanden ist.

In Figur 3 ist das in der Figur 2 veranschaulichte Verfahren nochmals in Form einer alternativen Darstellung veranschaulicht. Wie aus der Figur 3 ersichtlich, sind die Trenneinheiten 31, 35, 51 und 52 in entsprechenden Anlagen in Form von Destillationssäulen realisiert.

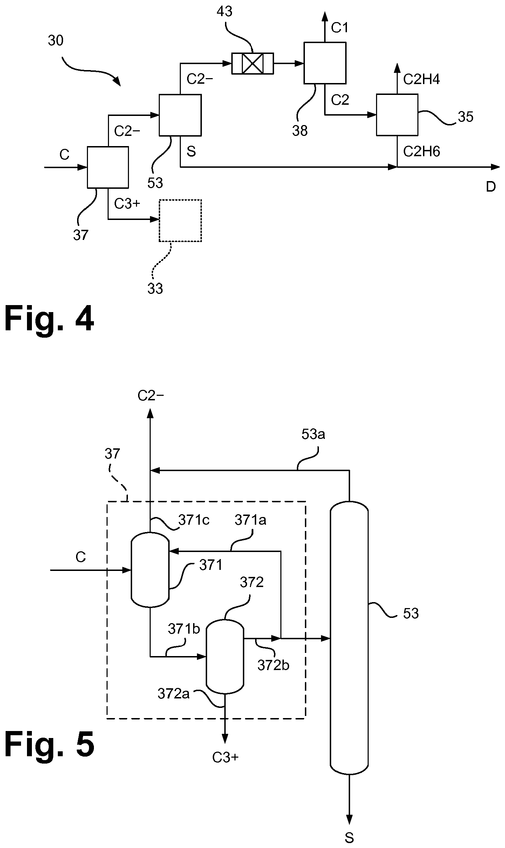

In einer wiederum der Figur 2 entsprechenden Darstellung zeigt die Figur 4 ein nicht erfindungsgemäßes Vergleichsbeispiel, bei der die Trennung des Kohlenwasserstoffgemischs bzw. des Spaltgases C nach dem Prinzip "Deethanizer First" erfolgt. Dies bedeutet, wie erläutert, dass in einer dort vorgesehenen ersten Trenneinheit 37 aus dem Spaltgas C eine Fraktion gebildet wird, die den überwiegenden Anteil der in dem Spaltgas C enthaltenen Kohlenwasserstoffe mit zwei oder weniger Kohlenstoffatomen enthält. Diese C2minus-Fraktion enthält jedoch beim Dampfspalten ethanreicher(er) Frischeinsätze ebenso hohe Gehalte an Ethan wie zuvor, die gegebenenfalls in herkömmlichen Trennanlagen nicht toleriert werden können, wenn diese zur Trennung von Spaltgasen eingerichtet sind, die durch Dampfspalten ethanarmer Frischeinsätze erhalten werden.

Eine zur Verarbeitung der C3plus-Kohlenwasserstoffe eingerichtete Trenneinheit 33 ist hier nur schematisch veranschaulicht, kann jedoch, neben nachgeordneten Einrichtungen, ebenfalls vorhanden sein.

Auch wird daher zunächst in einer zusätzlichen Trenneinheit 53 eine Ethan enthaltende Fraktion S aus der ersten Fraktion, hieraus der zunächst vorliegenden C2minus-Fraktion stromab der Trenneinheit 37, abgetrennt, und zwar in einer Menge, die den Ethangehalt in der verbleibenden C2minus-Fraktion stromab der Trenneinheit 53 auf eine tolerierbare Menge, z.B. weniger als 25%, reduziert. Anders als zuvor enthält diese Ethan enthaltende Fraktion hier jedoch keine nennenswerten Mengen an schwereren Verbindungen, d.h. keine C3plus-Kohlenwasserstoffe.

Die C2minus-Fraktion wird, wie bereits in Figur 1 gezeigt, einer Trenneinheit 38 zugeführt, die wie zu Figur 2B erläutert arbeitet. Aufgrund der vorgeschalteten Trenneinheit 53 muss diese jedoch eine deutlich geringere Ethanmenge verarbeiten.

Die Hydriereinheit 43 wird vorzugsweise wie in der Figur 4 dargestellt angeordnet (vgl. abweichende Anordnung gegenüber Figur 1), so dass sie nur den C2minus-Strom stromab der zusätzlichen Trenneinheit 53 verarbeiten muss.

Alternativ zu der in der Figur 4 dargestellten Anordnung der zusätzlichen Trenneinheit 53 stromab der Trenneinheit 37 kann diese auch beispielsweise parallel zur Trenneinheit 37 oder stromab der Trenneinheit 38 zur Bearbeitung des dort anfallenden C2-Stroms vorgesehen sein.

In Figur 5 ist die erstgenannte Alternative als ein nicht erfindungsgemäßes Vergleichsbeispiel dargestellt, nämlich die parallele Anordnung zu der Trenneinheit 37. Die Trenneinheit 37 ist zusammen mit der zusätzlichen Trenneinheit 53 im Detail veranschaulicht.

Die Trenneinheit 37 umfasst im dargestellten Beispiel eine erste Trenneinrichtung 371 und eine zweite Trenneinrichtung 372 (auch als Doppelsäule bezeichnet).

Der ersten Trenneinrichtung 371 wird das Spaltgas C zugeführt. Die erste Trenneinrichtung 371 wird unter Verwendung eines flüssigen Rücklaufs 371a betrieben. Im Sumpf der ersten Trenneinrichtung 371 scheidet sich ein Sumpfprodukt ab, das im Wesentlichen noch sämtliche Komponenten des Spaltgases C enthält. Dieses wird als Strom 371b abgezogen und in die zweite Trenneinrichtung 372 eingespeist. Am Kopf der ersten Trenneinrichtung 371 wird ein Kopfstrom 371c abgezogen, bei dem es sich um einen C2minus-Strom handelt, dessen Ethangehalt unter anderem über die Menge des Rücklaufs 371a eingestellt werden kann.

In der zweiten Trenneinrichtung 372 wird ein Sumpfprodukt 372a in Form einer an anderen Kohlenwasserstoffen armen oder freien C3plus-Fraktion erhalten. In einem oberen Bereich dieser zweiten Trenneinrichtung 372 wird ein Strom 372b abgezogen und teilweise (nach Kondensation in einem nicht dargestellten Kondensator) als der flüssige Rücklauf 371a auf die erste Trenneinrichtung 371 aufgegeben und teilweise in die Trenneinheit 53 überführt, die beispielsweise als Destillationssäule wie zuvor erläutert ausgebildet ist.

Die Trenneinheit 53 ist, wie bereits zuvor erläutert, dazu ausgebildet, die Ethan enthaltende Fraktion S abzutrennen. Bei der Ethan enthaltenden Fraktion S (die ggf. noch Reste an C3plus-Kohlenwasserstoffen enthalten kann) handelt es sich um das Sumpfprodukt dieser Trenneinheit 53. Vom Kopf dieser Trenneinheit wird hingegen ein Strom 53a abgezogen, der neben Ethan (das nicht im Sumpf der Trenneinheit 53 abgeschieden wird) auch andere Kohlenwasserstoffe mit drei Kohlenstoffatomen enthält. Dieser Strom kann damit mit dem am Kopf der ersten Trenneinrichtung 371 abgezogenen Kopfstrom 371c vereinigt werden.

Die vereinigten Ströme 53a und 371c entsprechen einem C2minus-Strom, wie er gemäß Figur 4 in der Trenneinheit 53 erhalten wird.

## Patentansprüche

1. Verfahren, bei dem ein Kohlenwasserstoffgemisch (Bezugszeichen C) zumindest teilweise durch Dampfspalten (10) gewonnen wird, das zumindest Kohlenwasserstoffe mit einem, zwei und drei Kohlenstoffatomen, darunter Ethan und Ethylen, enthält, wobei aus dem Kohlenwasserstoffgemisch (C) zunächst unter zumindest teilweiser Abtrennung anderer Komponenten eine erste Fraktion (Bezugszeichen C2+) gewonnen wird, die den überwiegenden Teil der in dem Kohlenwasserstoffgemisch (C) enthaltenen Kohlenwasserstoffe mit zwei und mehr Kohlenstoffatomen enthält, wobei aus der ersten Fraktion (C2+) anschließend weitere Fraktionen (Bezugszeichen C2, C2H4, C3+, C2H6) gewonnen werden, darunter Ethan (Bezugszeichen C2H4), das zum Dampfspalten (10) zurückgeführt wird, und Ethylen (Bezugszeichen C2H4), **dadurch gekennzeichnet, dass** nach der zumindest teilweisen Abtrennung der anderen Komponenten und vor der Gewinnung der weiteren Fraktionen (C2, C2H4, C3+, C2H6) aus dem Kohlenwasserstoffgemisch (C) ferner eine Ethan enthaltende Fraktion (Bezugszeichen R) in einer Menge abgetrennt wird, die den Ethangehalt in der ersten Fraktion (C2+) auf weniger als 25% verringert, wobei die Ethan enthaltende Fraktion (R)höchstens 10% auf molarer Basis an anderen Kohlenwasserstoffen mit zwei Kohlenstoffatomen enthält.

2. Verfahren nach Anspruch 1, bei dem der Gehalt an Ethan in der ersten Fraktion (C2+) durch das Abtrennen der Ethan enthaltenden Fraktion (R) auf einen Ethangehalt von weniger als 20%, weniger als 17%, weniger als 15%, weniger als 13% oder weniger als 10% verringert wird.

3. Verfahren nach einem der vorstehenden Ansprüche, bei dem die Ethan enthaltende Fraktion (R) weniger als 1,5%, weniger als 1,0%, weniger als 5000 ppm, weniger als 4000 ppm oder weniger als 3000 ppm Ethylen enthält.

4. Verfahren nach Anspruch 2 oder 3, bei dem die Ethan enthaltende Fraktion (R) zumindest teilweise durch Dampfspalten (10) bearbeitet wird.

5. Verfahren nach einem der vorstehenden Ansprüche, bei dem zur Gewinnung der ersten Fraktion (C2+) aus dem Kohlenwasserstoffgemisch (C) eine Fraktion (C1-) abgetrennt wird, die den überwiegenden Anteil des zuvor in dem Kohlenwasserstoffgemisch (C) enthaltenen Methans und Wasserstoffs enthält, so dass in der ersten Fraktion (C2) der überwiegende Anteil der zuvor in dem Kohlenwasserstoffgemisch (C) enthaltenen Kohlenwasserstoffe mit zwei und mehr Kohlenstoffatomen enthalten ist.

6. Verfahren nach einem der vorstehenden Ansprüche, bei dem das Kohlenwasserstoffgemisch (C) oder eine hiervon abgeleitete Fraktion (C2+) einem Hydrierverfahren unterzogen wird.

## Claims

1. Method in which a hydrocarbon mixture (reference sign C) is obtained at least partially via steam cracking (10), which mixture contains at least hydrocarbons having one, two, and three carbon atoms, among them ethane and ethylene, wherein a first fraction (reference sign C2+) is obtained from the hydrocarbon mixture (C), initially with at least partial separation of other components, which first fraction contains the predominant portion of the hydrocarbons having two or more carbon atoms contained in the hydrocarbon mixture (C); wherein further fractions (reference signs C2, C2H4, C3+, C2H6) are then obtained from the first fraction (C2+), among them ethane (reference sign C2H4), which is returned to the steam cracking (10), and ethylene (reference sign C2H4); **characterized in that**, after the at least partial separation of the other components and prior to obtaining the further fractions (C2, C2H4, C3+, C2H6) from the hydrocarbon mixture (C), an ethane-containing fraction (reference sign R) is also separated in an amount which reduces the ethane content in the first fraction (C2+) to less than 25%, wherein the ethane-containing fraction (R) contains at most 10% on a molar basis of other hydrocarbons having two hydrocarbon atoms.

2. Method according to claim 1, in which the ethane content in the first fraction (C2+) is reduced, by separating the ethane-containing fraction (R), to an ethane content of less than 20%, less than 17%, less than 15%, less than 13%, or less than 10%.

3. Method according to any one of the preceding claims, in which the ethane-containing fraction (R) contains less than 1.5%, less than 1.0%, less than 5000 ppm, less than 4000 ppm, or less than 3000 ppm ethylene.

4. Method according to claim 2 or 3, in which the ethane-containing fraction (R) is at least partially processed via steam cracking (10).

5. Method according to any one of the preceding claims, in which, in order to obtain the first fraction (C2+) from the hydrocarbon mixture (C), a fraction (C1-), which contains the predominant portion of the methane and hydrogen previously contained in the hydrocarbon mixture (C), is separated so that the predominant portion of the hydrocarbons having two or more carbon atoms that was previously contained in the hydrocarbon mixture (C) is contained in the first fraction (C2).

6. Method according to any one of the preceding claims, in which the hydrocarbon mixture (C) or a fraction (C2+) derived therefrom is subjected to a hydrogenation process.

## Revendications

1. Procédé, selon lequel un mélange d'hydrocarbures (référence C) est obtenu au moins partiellement par craquage à la vapeur (10), ledit mélange contient au moins des hydrocarbures ayant un, deux et trois atomes de carbone, parmi lesquels de l'éthane et de l'éthylène, où, à partir du mélange d'hydrocarbures (C), par séparation au moins partielle d'autres composants, une première fraction (référence C2+) est d'abord obtenue, laquelle contient la partie essentielle des hydrocarbures ayant au moins deux atomes de carbone contenus dans le mélange d'hydrocarbures (C), où, à partir de la première fraction (C2+), d'autres fractions (références C2, C2H4, C3+, C2H6) sont ensuite obtenues parmi lesquelles de l'éthane (référence C2H4), lequel est réintroduit dans le craquage à la vapeur (10), et de l'éthylène (référence C2H4), **caractérisé en ce que**, après la séparation au moins partielle des autres composants et avant l'obtention des autres fractions (C2, C2H4, C3+, C2H6) du mélange d'hydrocarbures (C), une fraction contenant de l'éthane (référence R) est en outre séparée en une quantité, laquelle diminue la teneur en éthane dans la première fraction (C2+) à moins de 25 %, où la fraction contenant de l'éthane (R) contient au plus 10 % sur une base molaire d'autres hydrocarbures ayant deux atomes de carbone.

2. Procédé selon la revendication 1, selon lequel la teneur en éthane dans la première fraction (C2+) est diminuée par séparation de la fraction contenant de l'éthane (R) à une teneur en éthane inférieure à 20 %, inférieure à 17 %, inférieure à 15 %, inférieure à 13 % ou inférieure à 10 %.

3. Procédé selon l'une quelconque des revendications précédentes, selon lequel la fraction contenant de l'éthane (R) contient moins de 1,5 %, moins de 1,0 %, moins de 5 000 ppm, moins de 4 000 ppm ou moins de 3 000 ppm d'éthylène.

4. Procédé selon la revendication 2 ou 3, selon lequel la fraction contenant de l'éthane (R) est traitée au moins partiellement par craquage à la vapeur (10).

5. Procédé selon l'une quelconque des revendications précédentes, selon lequel, pour l'obtention de la première fraction (C2+) à partir du mélange d'hydrocarbures (C), une fraction (C1-) est séparée, laquelle contient la proportion essentielle du méthane et de l'hydrogène contenus au préalable dans le mélange d'hydrocarbures (C) de telle sorte que dans la première fraction (C2) la proportion essentielle des hydrocarbures ayant au moins deux atomes de carbone contenus au préalable dans le mélange d'hydrocarbures (C) est contenue.

6. Procédé selon l'une quelconque des revendications précédentes, selon lequel le mélange d'hydrocarbures (C) ou une fraction (C2+) dérivée de celui-ci est soumis(e) à un procédé d'hydrogénation.
